# EUROPEAN PATENT APPLICATION

(11) **EP 4 360 745 A1**
(43) Date of publication of application: **01.05.2024**
(21) Application number: 23163498.1
(22) Date of filing: 22.03.2023
(51) Int. Cl.: B01F 23/41, B01F 25/431, B01F 33/30, B01F 33/81, B01F 35/10, B01F 35/71, B01J 19/24

(54) **NANOPARTICLE PREPARATION**

(30) Priority: 26.10.2022 US 202217973656; 24.10.2022 CN 202211303843
(71) Applicant: Beijing Jitai Pharmaceutical Technology Co., Ltd., Beijing 100192 (CN); Hangzhou Jitai Pharmaceutical Technology Co., Ltd., Hangzhou, Zhejiang 310051 (CN)
(72) Inventor: LIU, Andong, ZHEJIANG, 310051 (CN); YANG, Liu, ZHEJIANG, 310051 (CN); JIANG, Tian, BEIJING, 100192 (CN); LAI, Caida, ZHEJIANG, 310051 (CN); WANG, Wenshou, ZHEJIANG, 310051 (CN)
(74) Representative: Cabinet Becker et Associés

(57) **Abstract**

A nanoparticle preparation system, a control method of the nanoparticle preparation system, an electronic device, a non-transitory computer readable medium, and a method for preparing nanoparticles using the nanoparticle preparation system are disclosed. The nanoparticle preparation system includes a mixing element, a first liquid suction device and a second liquid suction device. The mixing element includes a plurality of mixing channels; the first liquid suction device includes a plurality of first liquid suction tubes arranged in rows along a first direction and configured to input a first preparation solution into the plurality of mixing channels through first inlets of the mixing channels, respectively; and the second liquid suction device includes a plurality of second liquid suction tubes arranged in rows along a second direction and configured to input a second preparation solution into the plurality of mixing channels through second inlets of the mixing channels, respectively.

## Description

### TECHNICAL FIELD

Embodiments of the present disclosure relate to a nanoparticle preparation system, a control method of the nanoparticle preparation system, an electronic device, a non-transitory computer readable medium and a method for preparing nanoparticles by using the nanoparticle preparation system.

### BACKGROUND

In recent years, with the gradual deepening of human research in the field of genes, nucleic acid drugs (including DNA, RNA, gene editing tools, etc.) include developed rapidly. At present, using a chemical material as a carrier to encapsulate a nucleic acid drug to form nanoparticles has been widely used in a field of drug research and development and clinical medicine.

How to produce nanoparticles with controllable size, uniform size, regular morphology, high drug loading, stability and little difference between different batches largely depends on the preparation process parameters. Nanoparticles are often composed of a variety of components, apart from a great variety of nanomaterials themselves, there are many possibilities for the combination of nanoparticles due to varieties and addition ratios of multiple auxiliary components. Taking the nano-preparation containing four components as an example, adjusting the component concentration by 1 mol% as an increment, assuming that the components of four lipid molecules (A, B, C, D) remain unchanged, A%, B%, C% and D% are all integers, and A mol%+B mol%+C mol%+D mol% = 100, there are 7,153 combinations. If one of the components is screened in this case (assuming that it is A and there are 100 candidate molecules), there are 105 combinations. In addition, changes such as the ratio of nitrogen to phosphorus, the concentration of chemical materials, the concentration of drugs, the acidity and alkalinity of the buffer used and the ionic strength, etc. all directly affect the effectiveness and targeting of nanoparticles, so it is also needed to screen them. The number of such screening is huge, which obviously needs to be screened by high- flux methods.

Therefore, a high-flux nanoparticle preparation system and method are needed to meet the requirements of screening nanoparticle formulations.

### SUMMARY

At least one embodiment of the present disclosure provides a nanoparticle preparation system, which includes a mixing element, a first liquid suction device and a second liquid suction device. The mixing element includes a plurality of mixing channels, the plurality of mixing channels are independent from each other and each of the plurality of mixing channels includes a first inlet, a second inlet and an outlet; the first liquid suction device includes a plurality of first liquid suction tubes arranged in rows along a first direction and configured to input a first preparation solution into the plurality of mixing channels through first inlets of the plurality of mixing channels, respectively; and the second liquid suction device includes a plurality of second liquid suction tubes arranged in rows along a second direction and configured to input a second preparation solution into the plurality of mixing channels through second inlets of the plurality of mixing channels, respectively.

For example, in some embodiments, the first liquid suction device further includes a plurality of first liquid suction pumps respectively connected to the plurality of first liquid suction tubes and configured to input the first preparation solution to corresponding mixing channels through corresponding first liquid suction tubes; the second liquid suction device further includes a plurality of second liquid suction pumps respectively connected to the plurality of second liquid suction tubes and configured to input the second preparation solution into corresponding mixing channels through corresponding second liquid suction tubes.

For example, in some embodiments, the nanoparticle preparation system further includes: a first preparation solution reservoir, including first solution accommodating wells arranged in an array in the first direction and a third direction perpendicular to the first direction for storing the first preparation solution; a second preparation solution reservoir, including second solution accommodating wells arranged in an array in the second direction and a fourth direction perpendicular to the second direction for storing the second preparation solution; the plurality of liquid suction tubes are movable in the third direction relative to the first preparation solution reservoir to sequentially input the first preparation solution in a plurality of rows of first solution accommodating wells into the plurality of mixing channels, respectively, the plurality of liquid suction tubes are movable in the fourth direction relative to the second preparation solution reservoir to sequentially input the second preparation solution in a plurality of rows of second solution accommodating wells into the plurality of mixing channels, respectively.

For example, in some embodiments, the first direction is the same as the second direction, the third direction is the same as the fourth direction, the plurality of first liquid suction tubes and the plurality of second liquid suction tubes are configured to move synchronously in the third direction and the fourth direction.

For example, in some embodiments, a number of the plurality of first liquid suction tubes and a number of the first solution accommodating wells in the first direction are the same as a number of the plurality of mixing channels, and a number of the second liquid suction tubes and a number of the second solution accommodating wells in the second direction are the same as the number of the mixing channels.

For example, in some embodiments, the nanoparticle preparation system further includes: a liquid outlet device, including a plurality of liquid outlet tubes arranged in rows along a fifth direction and configured to respectively guide nanoparticle products at outlets of the plurality of mixing channels out of the plurality of mixing channels.

For example, in some embodiments, the nanoparticle preparation system further includes: a product reservoir, including product accommodating wells arranged in an array in the fifth direction and a sixth direction perpendicular to the fifth direction; the liquid outlet device is movable in the sixth direction relative to the product reservoir to sequentially guide the nanoparticle products at the outlets of the plurality of mixing channels into a plurality of rows of product accommodating wells.

For example, in some embodiments, the nanoparticle preparation system further includes: a first cleaning liquid reservoir for storing cleaning liquid; and a second cleaning liquid reservoir for storing cleaning liquid, the first cleaning liquid reservoir is arranged at a side of the first preparation solution reservoir in the third direction, so that the plurality of first liquid suction tubes can move in the third direction to be communicated with the first cleaning liquid reservoir, thereby respectively inputting the cleaning liquid into the plurality of mixing channels through the first inlets; the second cleaning liquid reservoir is arranged at a side of the second preparation solution reservoir in the fourth direction, so that the plurality of second liquid suction tubes can move in the fourth direction to be communicated with the second cleaning liquid reservoir, thereby respectively inputting the cleaning liquid into the plurality of mixing channels through the second inlets.

For example, in some embodiments, the plurality of first liquid suction tubes can move in a vertical direction relative to the first preparation solution reservoir, the plurality of second liquid suction tubes can move in the vertical direction relative to the second preparation solution reservoir, the vertical direction is perpendicular to the first direction and the fourth direction.

For example, in some embodiments, the mixing element includes at least one microfluidic chip, and the plurality of mixing channels are arranged in the at least one microfluidic chip.

For example, in some embodiments, the first preparation solution reservoir further includes a first temperature control device configured to change a temperature of the first preparation solution to a first preparation temperature, and the second preparation solution reservoir further includes a second temperature control device configured to change a temperature of the second preparation solution to a second preparation temperature.

At least one embodiment of the present disclosure provides a control method of a nanoparticle preparation system, the nanoparticle preparation system includes a mixing element, a first liquid suction device and a second liquid suction device. The mixing element including a plurality of mixing channels, wherein the plurality of mixing channels are independent from each other and each of the plurality of mixing channels includes a first inlet, a second inlet and an outlet; the first liquid suction device, including a plurality of first liquid suction tubes arranged in rows along a first direction; and the second liquid suction device including a plurality of second liquid suction tubes arranged in rows along a second direction, the control method includes: obtaining a control condition; and executing an input operation according to the control condition, including: controlling the plurality of first liquid suction tubes of the first liquid suction device to input a first preparation solution into the plurality of mixing channels through first inlets of the plurality of mixing channels, respectively; and controlling the plurality of second liquid suction tubes of the second liquid suction device to input a second preparation solution into the plurality of mixing channels through second inlets of the plurality of mixing channels, respectively.

For example, in some embodiments, the nanoparticle preparation system further includes: a first preparation solution reservoir, including first solution accommodating wells arranged in an array in the first direction and a third direction perpendicular to the first direction for storing the first preparation solution; and a second preparation solution reservoir, including second solution accommodating wells arranged in an array in the second direction and a fourth direction perpendicular to the second direction for storing the second preparation solution, the input operation includes: controlling the plurality of first liquid suction tubes of the first liquid suction device to move in the third direction relative to the first preparation solution reservoir, and sequentially inputting the first preparation solution in a plurality of rows of first solution accommodating wells into the plurality of mixing channels, respectively; and controlling the plurality of second liquid suction tubes of the second liquid suction device to move in the fourth direction relative to the second preparation solution reservoir, and sequentially inputting the second preparation solution in a plurality of rows of second solution accommodating wells into the plurality of mixing channels, respectively.

For example, in some embodiments, the nanoparticle preparation system further includes: a liquid outlet device, including a plurality of liquid outlet tubes arranged in rows along a fifth direction; and a product reservoir, including product accommodating wells arranged in an array in the fifth direction and a sixth direction perpendicular to the fifth direction, the control method further includes executing an output operation, the output operation includes: controlling the plurality of liquid outlet tubes of the liquid outlet device to move in the sixth direction relative to the product reservoir, and sequentially guiding nanoparticle products at the outlets of the plurality of mixing channels into a plurality of rows of product accommodating wells.

For example, in some embodiments, the nanoparticle preparation system further includes: a first cleaning liquid reservoir for storing cleaning liquid, the first cleaning liquid reservoir being arranged at a side of the first preparation solution reservoir along a third direction, a second cleaning liquid reservoir for storing cleaning liquid, the second cleaning liquid reservoir being arranged at a side of the second preparation solution reservoir along a fourth direction, the control method further includes executing a cleaning operation, the cleaning operation includes: controlling the plurality of first liquid suction tubes of the first liquid suction device to move to the first cleaning liquid reservoir in the third direction, and respectively inputting the cleaning liquid in the first cleaning liquid reservoir into the plurality of mixing channels; and controlling the plurality of second liquid suction tubes of the second liquid suction device to move to the second cleaning liquid reservoir in the fourth direction, and respectively inputting the cleaning liquid in the second cleaning liquid reservoir into the plurality of mixing channels, the cleaning operation is executed after each input operation.

For example, in some embodiments, the control condition includes a mixing speed and a mixing ratio of the first preparation solution and the second preparation solution; the control method includes: determining a first flow rate of the first preparation solution and a second flow rate of the second preparation solution according to the mixing speed and the mixing ratio; in the input operation: controlling the plurality of first liquid suction tubes of the first liquid suction device to input the first preparation solution into the plurality of mixing channels at the first flow rate through first inlets of the plurality of mixing channels, respectively; and controlling the plurality of second liquid suction tubes of the second liquid suction device to input the second preparation solution into the plurality of mixing channels at the second flow rate through second inlets of the plurality of mixing channels, respectively.

For example, in some embodiments, the control condition further includes preparation temperature, the control method includes: controlling a first temperature control device of the first preparation solution reservoir to change a temperature of the first preparation solution to a first preparation temperature according to the preparation temperature; and controlling a second temperature control device of the second preparation solution reservoir to change a temperature of the second preparation solution to a second preparation temperature according to the preparation temperature.

At least one embodiment of the present disclosure provides an electronic device, which includes: a processor; and a memory storing instructions, the processor execute the abovementioned control method of the nanoparticle preparation system according upon the instructions being executed by the processor.

At least one embodiment of the present disclosure provides a non-transitory computer readable medium, storing instructions that can be executed by one or more computing devices, the execution of the instructions by the computing devices causes the computing devices to execute the abovementioned control method of the nanoparticle preparation system.

At least one embodiment of the present disclosure provides a method for preparing nanoparticles by using a nanoparticle preparation system, including the following steps: providing a mixing element, including a plurality of mixing channels, the plurality of mixing channels are independent from each other and each of the plurality of mixing channels includes a first inlet, a second inlet and an outlet; providing a first liquid suction device, including a plurality of first liquid suction tubes arranged in rows along a first direction; providing a second liquid suction device including a plurality of second liquid suction tubes arranged in rows along a second direction; and performing an input operation according to a control condition, the input operation includes: inputting a first preparation solution into the plurality of mixing channels through first inlets of the plurality of mixing channels by using the plurality of first liquid suction tubes of the first liquid suction device; and inputting second preparation solution into the plurality of mixing channels through the second inlets of the plurality of mixing channels by using the plurality of second liquid suction tubes of the second liquid suction device.

For example, in some embodiments, the method further includes: providing a first preparation solution reservoir including first solution accommodating wells arranged in an array in the first direction and a third direction perpendicular to the first direction; providing a second preparation solution reservoir including second solution accommodating wells arranged in an array in the second direction and a fourth direction perpendicular to the second direction; and the input operation includes: using the plurality of first liquid suction tubes of the first liquid suction device to move in a third direction relative to the first preparation solution reservoir, and sequentially inputting the first preparation solution in a plurality of rows of first solution accommodating wells into the plurality of mixing channels; and using the plurality of second liquid suction tubes of the second liquid suction device to move in a fourth direction relative to the second preparation solution reservoir, and sequentially inputting the second preparation solution in a plurality of rows of second solution accommodating wells into the plurality of mixing channels.

For example, in some embodiments, the control condition includes a mixing speed and a mixing ratio of the first preparation solution and the second preparation solution; the method includes: determining a first flow rate of the first preparation solution and a second flow rate of the second preparation solution according to the mixing speed and the mixing ratio; in the input operation: using the plurality of first liquid suction tubes of the first liquid suction device to input the first preparation solution into the mixing channels at the first flow rate through the first inlets of the plurality of mixing channels, respectively; and using the plurality of second liquid suction tubes of the second liquid suction device to input the second preparation solution into the mixing channels at the second flow rate through the second inlets of the plurality of mixing channels, respectively.

For example, in some embodiments, the control condition further includes a preparation temperature, the method includes: using a first temperature control device of the first preparation solution reservoir to change a temperature of the first preparation solution to a first preparation temperature; and using a second temperature control device of the second preparation solution reservoir to change a temperature of the second preparation solution to a second preparation temperature.

For example, in some embodiments, the method further includes: providing a liquid outlet device including a plurality of liquid outlet tubes arranged in rows along a fifth direction; providing a product reservoir including a plurality of product accommodating wells arranged in an array in the fifth direction and a sixth direction perpendicular to the fifth direction; and performing an output operation, the output operation includes: using the plurality of liquid outlet tubes of the liquid outlet device to move in the sixth direction relative to the product reservoir, and sequentially guiding nanoparticle products at the outlets of the plurality of mixing channels to a plurality of rows of product accommodating wells.

For example, in some embodiments, the method further includes: providing a first cleaning liquid reservoir to a side of the first preparation solution reservoir in a first direction, the first cleaning liquid reservoir stores cleaning liquid; providing a second cleaning liquid reservoir to a side of the second preparation solution reservoir in a first direction, the second cleaning liquid reservoir stores cleaning liquid; and performing a cleaning operation, the cleaning operation includes: moving the plurality of first liquid suction tubes of the first liquid suction device to the first cleaning liquid reservoir in a third direction relative to the first preparation solution reservoir and the first cleaning liquid reservoir, and inputting the cleaning liquid in the first cleaning liquid reservoir into the plurality of mixing channels, respectively; and moving the plurality of second liquid suction tubes of the second liquid suction device to the second cleaning liquid reservoir in a fourth direction relative to the second preparation solution reservoir and the second cleaning liquid reservoir, and inputting the cleaning liquid in the second cleaning liquid reservoir into the plurality of mixing channels, respectively, the cleaning operation is performed after each input operation.

### BRIEF DESCRIPTION OF THE DRAWINGS

In order to more clearly explain the technical solutions of the embodiments of the present disclosure, the following drawings that need to be used in the embodiments will be briefly described. It should be understood that the following drawings only show some embodiments of the present disclosure, so they should not be regarded as limiting the scope. For those of ordinary skill in the art, other relevant drawings can be obtained according to these drawings without any creative effort.
Fig. 1 is a schematic top view of a nanoparticle preparation system according to an embodiment of the present disclosure;
Fig. 2 is a schematic diagram of a connection of a microfluidic chip with a liquid suction device and a liquid outlet device in a nanoparticle preparation system according to an embodiment of the present disclosure;
Fig. 3A is a schematic diagram of a mixing channel in a nanoparticle preparation system according to an embodiment of the present disclosure;
Fig. 3B is a schematic diagram of a mixing channel in a nanoparticle preparation system according to another embodiment of the present disclosure;
Fig. 4 is a schematic side view of a nanoparticle preparation system according to an embodiment of the present disclosure;
Fig. 5 is a partially enlarged top view of a first liquid suction device in a nanoparticle preparation system according to an embodiment of the present disclosure;
Fig. 6 is a partially enlarged top view of a second liquid suction device in a nanoparticle preparation system according to an embodiment of the present disclosure;
Fig. 7 is a partially enlarged top view of a liquid outlet device in a nanoparticle preparation system according to an embodiment of the present disclosure;
Fig. 8 is a perspective view of a platform of a nanoparticle preparation system according to an embodiment of the present disclosure;
Fig. 9 is a flowchart of a control method of a nanoparticle preparation system according to an embodiment of the present disclosure;
Fig. 10 is a schematic diagram of a hardware structure of an electronic device according to an embodiment of the present disclosure;
Fig. 11 is a flowchart of a method for preparing nanoparticles according to an embodiment of the present disclosure;
Fig. 12 is a schematic diagram of a volume accuracy of Daedalus of a sample prepared by a nanoparticle preparation system according to an embodiment of the present disclosure;
Fig. 13 is a schematic diagram of particle size and polymer dispersion index of nanoparticles for encapsulating siRNA (small interfering RNA) prepared by a non-automatic single-flux microfluidic hybrid system and a nanoparticle preparation system according to an embodiment of the present disclosure;
Fig. 14 is a schematic diagram of an encapsulation rate of nanoparticles for encapsulating siRNA (small interfering RNA) prepared by a non-automatic single-flux microfluidic hybrid system and a nanoparticle preparation system according to an embodiment of the present disclosure;
Fig. 15 is a schematic diagram of particle size and polymer dispersion index of nanoparticles for encapsulating mRNA (messenger RNA) prepared by a non-automatic single-flux microfluidic hybrid system and a nanoparticle preparation system according to an embodiment of the present disclosure;
Fig. 16 is a schematic diagram of an encapsulation rate of nanoparticles for encapsulating mRNA (messenger RNA) prepared by a non-automatic single-flux microfluidic hybrid system and a nanoparticle preparation system according to an embodiment of the present disclosure; and
Fig. 17 is a schematic diagram of in vivo delivery efficiency of nanoparticles for encapsulating mRNA (messenger RNA) prepared by a non-automatic single-flux microfluidic hybrid system and a nanoparticle preparation system according to an embodiment of the present disclosure.

### DETAILED DESCRIPTION

Hereinafter, a nanoparticle preparation system, a control method of the nanoparticle preparation system, an electronic device, a non-transitory computer readable medium, and a method for preparing nanoparticles using the nanoparticle preparation system according to embodiments of the present disclosure are described in detail with reference to the accompanying drawings. In order to make the purpose, technical resolutions and advantages of the utility present disclosure clearer, the technical resolutions in the embodiments of the present disclosure will be clearly and completely described below with reference to the accompanying drawings in the embodiments of the present disclosure, obviously, the described embodiments are a part of the embodiments of the present disclosure, but not all of them.

Therefore, the following detailed description of the embodiments of the present disclosure provided with reference to the accompanying drawings is not intended to limit the scope of the claimed disclosure, but only represents selected embodiments of the disclosure. Based on the embodiments in the present disclosure, all other embodiments obtained by ordinary technicians in this field without creative labor belong to the scope of protection of the present disclosure.

Unless otherwise defined by the context, singular forms include plural forms. Throughout this specification, the terms "comprising", "comprise", "including", and "include", etc. are used herein to specify the presence of the features, numbers, steps, operations, elements, components or combinations thereof, but do not exclude the presence or addition of one or more other features, numbers, steps, operations, elements, components or combinations thereof.

In addition, even though ordinal terms such as "first" and "second" are used to describe various components, these components are not limited by these terms, and these terms are only used to distinguish one element from other elements.

### Nanoparticle preparation system

At least one embodiment of the present disclosure provides a nanoparticle preparation system, the nanoparticle preparation system includes a mixing element including a plurality of mixing channels, the plurality of mixing channels are independent from each other and each of the plurality of mixing channels includes a first inlet, a second inlet and an outlet; a first liquid suction device including a plurality of first liquid suction tubes arranged in rows along a first direction and configured to input a first preparation solution into the plurality of mixing channels through first inlets of the plurality of mixing channels, respectively; and a second liquid suction device including a plurality of second liquid suction tubes arranged in rows along a second direction and configured to input a second preparation solution into the plurality of mixing channels through second inlets of the plurality of mixing channels, respectively.

Among particles that transport genes through chemical carriers, the chemical carrier that encapsulates foreign nucleic acid fragments is a structure composed of monomer molecules of the delivery material, which encapsulates and protects drugs to assemble into particles of 10 to 1000 nanometers. According to a size of chemical carriers, drug systems with nanometer particle diameter are called nano-drugs, and their types include lipid nanoparticles, liposomes, polymer nanoparticles, emulsifiers, organic or inorganic nanoparticles, and so on. There are many kinds of materials used to make nanoparticles, including lipids, polymers, polysaccharides, inorganic particles and surfactants, and so on.

There are many factors that affect the effect of nanocarriers, so it is needed to conduct high-flux screening.

The nanoparticle preparation system according to the embodiment of the present disclosure can be applied to high-flux screening of nanoparticle preparation and nanoparticle preparation. The nanoparticle preparation system has high-flux and can simultaneously prepare nanoparticles by using multiple mixing channels. In addition, the nanoparticle preparation system can also accurately control a mixing ratio and a mixing rate of each preparation solution, so as to achieve accurate control of preparation conditions. In addition, the nanoparticle preparation system can provide stable particle size and encapsulation efficiency of nanoparticles.

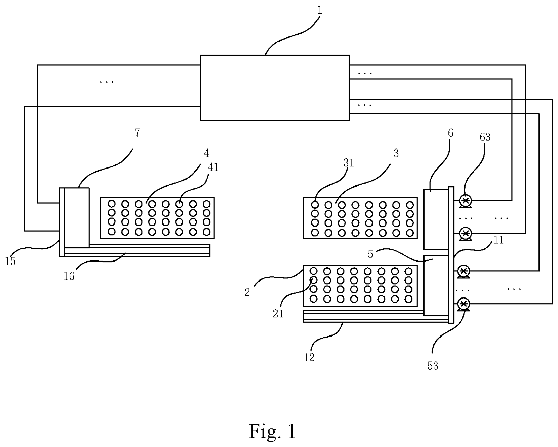

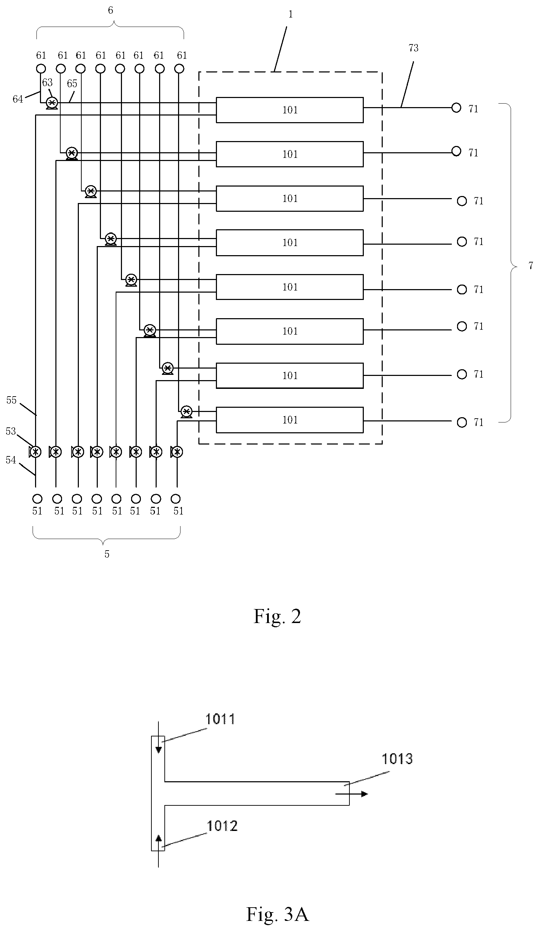

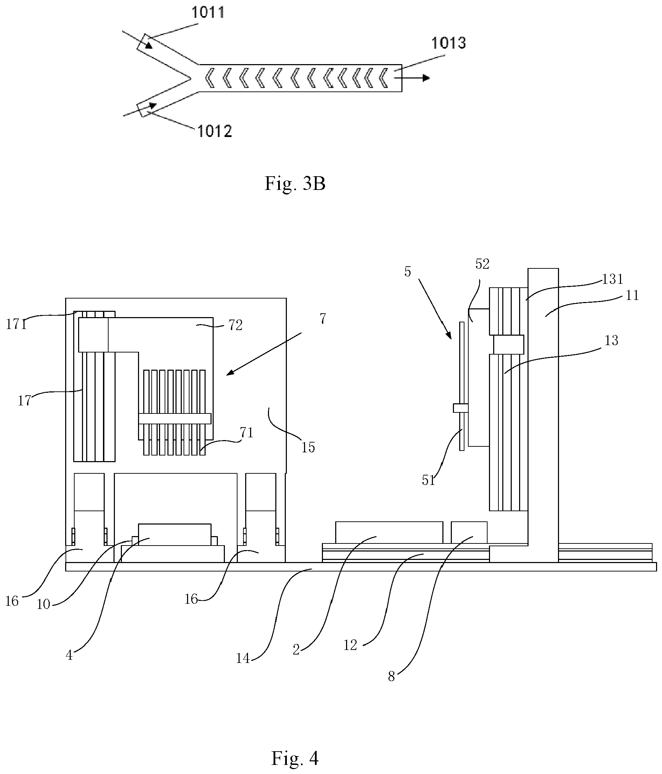

Fig. 1 is a schematic top view of a nanoparticle preparation system according to an embodiment of the present disclosure; Fig. 2 is a schematic diagram of a connection of a microfluidic chip with a liquid suction device and a liquid outlet device in a nanoparticle preparation system according to an embodiment of the present disclosure; Fig. 3A and Fig. 3B are schematic diagrams of mixing channels in a nanoparticle preparation system according to an embodiment of the present disclosure; and Fig. 4 is a schematic side view of a nanoparticle preparation system according to an embodiment of the present disclosure.

As illustrated by Fig. 1- Fig. 4, the nanoparticle preparation system includes a microfluidic chip 1 serving as the mixing element, a first liquid suction device 5, a second liquid suction device 6, a liquid outlet device 7, a first input well plate 2 serving as a first preparation solution reservoir, a second input well plate 3 serving as a second preparation solution reservoir, and an output well plate 4 as a product reservoir.

As illustrated by Fig. 2- Fig. 3B, the microfluidic chip 1 includes a plurality of mixing channels 101 which are independent from each other and include two inlets 1011, 1012 and an outlet 1013, respectively. Herein, the plurality of mixing channels 101 are independent from each other, which refers to that there is no fluid communication among the plurality of mixing channels 101. In the present example, the plurality of mixing channels 101 are combined into an integrated microfluidic chip 1. However, in other examples, the plurality of mixing channels 101 may not be included in the same microfluidic chip. For example, the plurality of mixing channels 101 can be included in separate microfluidic chips respectively. Alternatively, some of the plurality of mixing channels 101 are included in one microfluidic chip, while other mixing channels 101 are included in other one or more other microfluidic chips. The present disclosure is not limited thereto. In addition, the mixing element is not limited to the microfluidic chip, but may be other forms of elements including the mixing channels 101. Herein, the microfluidic chip refers to an element that includes channels in micron or nanometer scale.

A structure of each mixing channel 101 can be realized by using the existing single-flux mixing channels 101. Fig. 3A and Fig. 3B show schematic diagrams of mixing channels according to different embodiments of the present disclosure. As illustrated by Fig. 3A and Fig. 3B, each mixing channel 101 includes two inlets 1011, 1012 and one outlet 1013. A first preparation solution stored in the first input well plate 2 and a second preparation solution stored in the second input well plate 3 can be input into the mixing channels 101 through the two inlets 1011, 1012, respectively. By mixing the first preparation solution and the second preparation solution in the mixing channels 101, nanoparticles or other products can be generated. In the present example, the mixing channel 101 includes two inlets 1011 and 1012. However, in other examples, the mixing channel 101 may include more than two inlets, so that more preparation solution is input into the mixing channel 101 for preparing nanoparticles or other products. In addition, the mixing channel 101 may also include more than one outlet 1013. The present disclosure is not limited to the shape, arrangement, etc. of the mixing channel 101 as long as the preparation solution can be mixed to produce nanoparticles or other products.

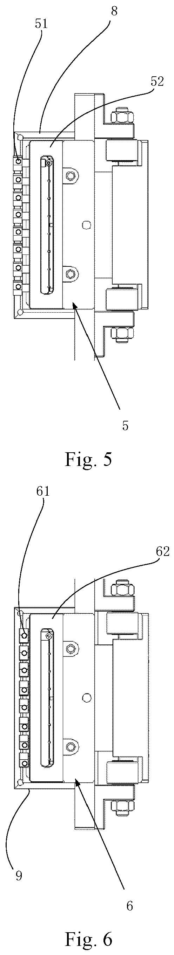

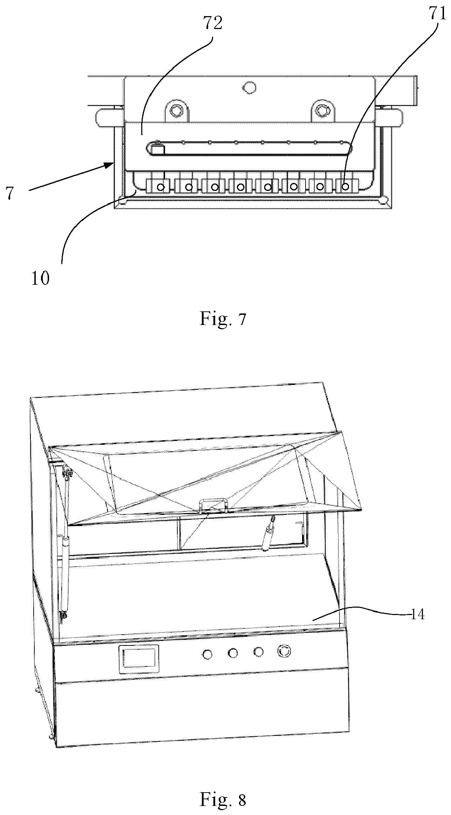

Fig. 5 is a partially enlarged top view of a first liquid suction device 5 in a nanoparticle preparation system according to an embodiment of the present disclosure, Fig. 6 is a partially enlarged top view of a second liquid suction device 6 in a nanoparticle preparation system according to an embodiment of the present disclosure, and Fig. 7 is a partially enlarged top view of a liquid outlet device 7 in the nanoparticle preparation system according to an embodiment of the present disclosure.

As illustrated by Fig. 2 and Fig. 4- Fig. 7, the first suction device 5 includes a plurality of first liquid suction tubes 51 arranged in rows, the second suction device 6 includes a plurality of second liquid suction tubes 61 arranged in rows, and the liquid outlet device 7 includes a plurality of liquid outlet tubes 71 arranged in rows. The plurality of first liquid suction tubes 51 respectively input the first preparation solution into the plurality of mixing channels 101 through the first inlets 1011 of the plurality of mixing channels 101. The plurality of second liquid suction tubes 61 respectively input the second preparation solution into the plurality of mixing channels 101 through the second inlets 1012 of the plurality of mixing channels 101. The plurality of liquid outlet tubes 71 respectively guide the nanoparticle products out of the mixing channels 101 through the outlets 1013 of the plurality of mixing channels 101. In addition, the first liquid suction device 5 further includes a plurality of first liquid suction pumps 53, which are connected to the plurality of first liquid suction tubes 51, respectively, and input the first preparation solution to the corresponding mixing channels 101 through the corresponding first liquid suction tubes 51. The second liquid suction device 6 further includes a plurality of second liquid suction pumps 63, which are connected to the plurality of second liquid suction tubes 61, respectively, and are configured to input the second preparation solution into the corresponding mixing channels 101 through the corresponding second liquid suction tubes 61.

The first input well plate 2 and the second input well plate 3 can be a 96-well plate or a 384-well plate, respectively. By adopting the standard 96-well plate or 384-well plate, the universality of the preparation system can be improved and the cost of the preparation system can be reduced. Correspondingly, the output well plate 4 can also be a 96-well plate or a 384-well plate.

The plurality of first liquid suction tubes 51 may be arranged along a first direction. The first input well plate 2 may be disposed below the plurality of first liquid suction tubes 51, and may be arranged to include first solution accommodating wells 21 arranged in an array in the first direction and a third direction perpendicular to the first direction for storing the first preparation solution. A number of the plurality of first liquid suction tubes 51 arranged along the first direction number may be equal to a number of a plurality of first solution accommodating wells 21 arranged along the first direction and may also be equal to a number of the mixing channels 101. Therefore, the plurality of liquid suction tubes 51 can simultaneously suck the first preparation solution in the plurality of first solution accommodating wells 21 arranged in rows along the first direction and input the first preparation solution into the mixing channels 101, respectively. As illustrated by Fig. 2, the first input tube 54 is connected between the first liquid suction tube 51 and the first suction pump 53, and the first output tube 55 is connected between the first liquid suction pump 53 and the first inlet 1011 of the mixing channel 101. The first liquid suction pump 53 is activated to input the first preparation solution from the first solution accommodating well 21 into the mixing channel 101 at a specific flow rate through the first liquid suction tube 51, the first input tube 54, the first liquid suction pump 53 and the first output tube 55.

The plurality of second liquid suction tubes 61 may be arranged along a second direction. The second input well plate 3 may be disposed below the plurality of second liquid suction tubes 61, and may be arranged to include second solution accommodating wells 31 arranged in an array in the second direction and a fourth direction perpendicular to the second direction for storing the second preparation solution. A number of the plurality of second liquid suction tubes 61 arranged along the second direction may be equal to a number of a plurality of second solution accommodating wells 31 arranged along the second direction and may also be equal to a number of the mixing channels 101. Therefore, the plurality of second liquid suction tubes 61 can simultaneously suck the second preparation solution in the plurality of second solution accommodating wells 31 arranged in rows along the second direction and input the second preparation solution into the mixing channels 101, respectively. As illustrated by Fig. 2, the second input tube 64 is connected between the second liquid suction tubes 61 and the second liquid suction pump 63, and the second output tube 65 is connected between the second liquid suction pump 63 and the second inlet 1012 of the mixing channel 101. The second liquid suction pump 63 is activated to input the second preparation solution from the second solution accommodating well 31 into the mixing channel 101 at a specific flow rate through the second liquid suction tube 61, the second input tube 64, the second liquid suction pump 63 and the second output tube 65.

A plurality of liquid outlet tubes 71 may be arranged along a fifth direction. The output well plate 4 may be disposed below the plurality of liquid outlet tubes 71, and may be arranged to include product accommodating wells 41 arranged in an array in the fifth direction and a sixth direction perpendicular to the fifth direction for storing products. A number of the plurality of liquid outlet tubes 71 arranged along the fifth direction may be equal to a number of a plurality of product accommodating wells 41 arranged along the fifth direction and may also be equal to a number of mixing channels 101. Therefore, the plurality of liquid outlet tubes 71 can simultaneously guide the products flowing out at the outlets 1013 of the mixing channels 101 into the plurality of product accommodating wells 41 arranged in rows along the fifth direction. As illustrated by Fig. 2, the output tube 73 is connected between the liquid outlet tube 71 and the outlet 1013 of the mixing channel 101. The nanoparticle product is input into the product accommodating well 41 through the output tube 73 and the liquid outlet tube 71.

In the present embodiment, a number of the mixing channels 101, a number of the first liquid suction tubes 51, a number of the second liquid suction tubes 61 and a number of the liquid outlet tubes 71 are all eight, but the present disclosure does not limit the number of the mixing channels 101, the number of the first liquid suction tubes 51, the number of the second liquid suction tubes 61 and the number of the liquid outlet tubes 71. The mixing channels 101, the first liquid suction tubes 51, the second liquid suction tubes 61 and the liquid outlet tubes 71 can be in other numbers, such as 2, 6 or 16, respectively.

In the present embodiment, a number of the first solution accommodating wells 21, a number of the second solution accommodating wells 31 and a number of the product accommodating wells 41 are 8 (in the first direction, second direction and fifth direction, respectively) ^{∗}12 (in the second direction, fourth direction and sixth direction, respectively), but the present disclosure does not limit the number of the first solution accommodating wells 21, the number of the second solution accommodating wells 31 and the number of the product accommodating wells 41. For example, the number of the first solution accommodating wells 21, the number of the second solution accommodating wells 31 and the number of the product accommodating wells 41 may be 2^{∗}8, 6^{∗}12, 16^{∗}24, etc. respectively.

The plurality of first liquid suction tubes 51 are movable in the third direction relative to the first input well plate 2 to sequentially input the first preparation solution in the rows of first solution accommodating wells 21 arranged along the first direction into the plurality of mixing channels 101, respectively. The plurality of second liquid suction tubes 61 can be moved in the fourth direction relative to the second input well plate 3 to sequentially input the second preparation solution in the rows of second solution accommodating wells 31 arranged along the third direction into the plurality of mixing channels 101, respectively. The plurality of liquid outlet tubes 71 can be moved in the sixth direction relative to the output well plate to sequentially transfer the products at the outlets 1013 of the plurality of mixing channels 101 into the rows of product accommodating wells 41 arranged in rows along the fifth direction.

For example, the plurality of first liquid suction tubes 51, the plurality of second liquid suction tubes 61 and the plurality of first liquid outlet tubes 71 can be moved synchronously. For example, the plurality of first liquid suction tubes 51 move in the third direction to be located directly above a first row of first solution accommodating wells 21, and respectively input the first preparation solution in the first row of first solution accommodating wells 21 into the mixing channels 101. At this time, the plurality of second liquid suction tubes 61 move in the fourth direction to be located directly above a first row of second solution accommodating wells 31, and respectively input the second preparation solution in the first row of second solution accommodating wells 31 into the mixing channels 101. And at this time, the plurality of first liquid outlet tubes 71 move in the sixth direction to be located directly above a first row of product accommodating wells 41, and transfer the products in the mixing channels 101 to the first row of product accommodating wells 41, respectively. Then, the plurality of first liquid suction tubes 51 move in the third direction to be located directly above a second row of first accommodating wells, and respectively input the first preparation solution in the second row of first solution accommodating wells 21 into the mixing channels 101. At this time, the plurality of second liquid suction tubes 61 move in the fourth direction to be located directly above a second row of second solution accommodating wells 31, and respectively input the second preparation solution in the second row of second solution accommodating wells 31 into the mixing channels 101. And at this time, the plurality of first liquid outlet tubes 71 move in the sixth direction to be located directly above a second row of product accommodating wells 41, and transfer the products in the mixing channels 101 to the second row of product accommodating wells 41, respectively. Then, the plurality of first liquid suction tubes 51 move in the third direction so as to be located directly above other rows of the first solution accommodating wells 21 in turn until the plurality of first liquid suction tubes 51 move directly above a last row of the first solution accommodating wells 21, and respectively input the first preparation solution in the last row of the first solution accommodating wells 21 into the mixing channels 101.At the same time, the plurality of second liquid suction tubes 61 move in the fourth direction so as to be located directly above other rows of the second solution accommodating wells 31 in turn until the plurality of second liquid suction tubes 61 move directly above a last row of the second solution accommodating wells 31, and respectively input the second preparation solution in the last row of the second solution accommodating wells 31 into the mixing channels 101. And at the same time, the plurality of first liquid outlet tubes 71 move in the sixth direction so as to be located directly above other rows of the product accommodating wells 41 in turn until the plurality of first liquid outlet tubes 71 move directly above a last row of the product accommodating wells 41, and transfer the products in the mixing channels 101 to the last row of product accommodating wells 41, respectively. Therefore, the product in the specific product accommodating well 41 is prepared through the specific mixing channel 101 by using the first preparation solution in the addressable first solution accommodating well 21 and the second preparation solution in the addressable second solution accommodating well 31. Parameters such as components and flow rates of the first and second preparation solutions, an internal structure of the mixing channel 101, and a temperature or the like can be changed to screen the preparation conditions of nanoparticles with high-flux or prepare nanoparticles with stable high-flux.

In the present embodiment, the first direction and the second direction are the same, the third direction and the fourth direction are the same, the first input well plate 2 and the second input well plate 3 are aligned with each other, and the plurality of first solution accommodating wells 21 and second solution accommodating wells 31 have the same number and are arranged in a same way. In addition, the plurality of first liquid suction tubes 51 and the plurality of second liquid suction tubes 61 can move synchronously in alignment with each other. Therefore, a motor and a guide rail can be used to simultaneously drive the plurality of first liquid suction tubes 51 and the plurality of second liquid suction tubes 61 to move in a same direction, thus saving the floor space of the system and reducing the cost of the system. As illustrated by Fig. 4 - Fig. 6, the plurality of first liquid suction tubes 51 are fixed to a first liquid suction bracket 52, and the plurality of second liquid suction tubes 61 are fixed to a second liquid suction bracket 62. The first liquid suction bracket 52 and the second liquid suction bracket 62 are fixed on a liquid suction total bracket 11. The liquid suction total bracket 11 is driven by a liquid suction horizontal driving device and moves back and forth along a liquid suction horizontal guide rail 12 fixed on a platform 14, so that the first liquid suction bracket 52 moves in the first direction and the second liquid suction n bracket 62 moves in the second direction which is the same as the first direction.

In the present embodiment, the fifth direction is perpendicular to the first direction and the second direction, and the sixth direction is the same as the third direction and the fourth direction. As illustrated by Fig. 4 and Fig. 7, the plurality of liquid outlet tubes 71 are fixed to a liquid outlet bracket 72. The liquid outlet bracket 72 is fixed to a liquid outlet total bracket 15, which is driven by a liquid outlet horizontal driving device and moves back and forth along a liquid outlet horizontal guide rail 16 fixed on the platform 14, so that the liquid outlet bracket 72 moves in the sixth direction.

The liquid suction horizontal driving device and the liquid outlet horizontal driving device can be realized by the existing horizontal driving mode, such as driving by a cylinder or a motor. Upon a cylinder being adopted to drive, two cylinders can be provided, one cylinder is fixed on the platform 14 as the liquid suction horizontal driving device, and then the liquid suction total bracket 11 is pushed to move back and forth along the liquid suction horizontal guide rail 12 by a piston rod moving back and forth. The other cylinder is fixed on the platform 14 as the liquid outlet horizontal driving device, and then the liquid outlet total bracket 15 is pushed to move back and forth along the liquid outlet horizontal guide rail 16 by a piston rod moving back and forth. It can also be driven by a motor with two motors. One motor is fixed to the liquid suction main bracket 11 as the liquid suction horizontal driving device. A rack is provided on the platform 14. The motor is provided with a gear meshed with the rack, and the rotation of the motor drives the liquid suction total bracket 11 to move back and forth along the liquid suction horizontal guide rail 12. The other motor is fixed to the liquid outlet total bracket 15 as the liquid outlet horizontal driving device, and a rack is arranged on the platform 14. The motor is provided with a gear meshed with the rack. The rotation of the motor drives the liquid outlet total bracket 15 to move back and forth along the liquid outlet horizontal guide rail 16.

It should be noted that the present disclosure is not limited to the movement of the first liquid suction tubes 51, the second liquid suction tubes 61 and the liquid outlet tubes 71. The first input well plate 2, the second input well plate 3 and the output well plate 4 can also be movable to realize the relative movement of the first liquid suction tubes 51 of the first liquid suction device 5 and the first input well plate 2, the relative movement of the second liquid suction tubes 61 of the second liquid suction device 6 and the second input well plate 3, and the relative movement of the liquid outlet tubes 71 of the liquid outlet device 7 and the output well plate 4.

In addition, the plurality of first liquid suction tubes 51 are movable in a vertical direction relative to the first input well plate 2 to insert and leave the first solution accommodating wells 21, the plurality of second liquid suction tubes 61 are movable in the vertical direction relative to the second input well plate 3 to insert and leave the second solution accommodating wells 31, and the plurality of liquid outlet tubes 71 are movable in the vertical direction relative to the output well plate 4 to insert and leave the product accommodating wells 41. The vertical direction is perpendicular to the first direction to the sixth direction extending on the horizontal plane.

For example, the first liquid suction bracket 52 is connected to a first vertical driving device, driven by the first vertical driving device, and moves up and down in the vertical direction along a first liquid suction vertical guide rail 13 fixed to the liquid suction total bracket 11, so as to move towards a direction where the first input well plate 2 is located or away from a direction where the first input well plate 2 is located. A first liquid suction vertical bracket fixed on the liquid suction total bracket 11 may be provided, and the first liquid suction vertical guide rail 13 is fixed to the first liquid suction vertical bracket. The second liquid suction bracket 62 is connected with the second vertical driving device, driven by the second vertical driving device, and moves up and down in the vertical direction along a second liquid suction vertical guide rail (not shown in the figure) fixed to the liquid suction total bracket 11, so as to move towards a direction where the second input well plate 3 is located or away from a direction where the second input well plate 3 is located. A second liquid suction vertical bracket fixed to the liquid suction total bracket 11 may be provided, and a second liquid suction vertical guide rail is fixed on the second liquid suction vertical bracket. The liquid outlet bracket 72 is connected with the vertical driving device, and is driven by the vertical driving device to move up and down along a liquid outlet vertical guide rail 17, so as to move towards a direction where the output well plate 4 is located or away from a direction where the output well plate 4 is located. A liquid outlet vertical bracket 171 fixed to the liquid outlet total bracket 15 may be provided, and the liquid outlet vertical guide rail 17 is fixed to the liquid outlet vertical bracket 171. The liquid outlet bracket 15 and the liquid outlet vertical guide rail 17 move back and forth along the liquid outlet horizontal guide rail 16 as a whole.

In addition, as illustrated by Fig. 1 and Fig. 4-Fig. 7, the nanoparticle preparation system further includes a first cleaning liquid reservoir 8, a second cleaning liquid reservoir 9 and a waste liquid reservoir 10. The first cleaning liquid reservoir 8 and the second cleaning liquid reservoir 9 are used for storing cleaning liquid.

One preparation cycle includes that the first liquid suction tubes 51 and the second liquid suction tubes 61 respectively suck the first preparation solution and the second preparation solution from the first solution accommodating wells 21 and the second solution accommodating wells 31, so as to input the first preparation solution and the second preparation solution into the mixing channels 101, and the liquid outlet tubes 71 transfers the products in the mixing channels 101 to the product accommodating wells 41. A cleaning operation can be performed between two preparation cycles. In the cleaning operation, the plurality of first liquid suction tubes 51 suck the cleaning liquid from the first cleaning liquid reservoir 8 and input the cleaning liquid into the mixing channels 101 through the first inlets 1011, and the plurality of second liquid suction tubes 61 suck the cleaning liquid from the second cleaning liquid reservoir 9 and input the cleaning liquid into the mixing channels 101 through the second inlets 1012 to clean the mixing channels 101. The first liquid suction pump 53 and the second liquid suction pump 63 respectively provide suction force for the suction of the first suction tubes 51 and the suction of the second suction tubes 61. In addition, in the cleaning operation, the plurality of liquid outlet tubes 71 transfer the cleaning liquid flowing out from the outlet 1013 of the mixing channels 101 to the waste liquid reservoir 10.

The first cleaning liquid reservoir 8 may be disposed at a side of the first preparation solution reservoir in the third direction, so that the plurality of first liquid suction tubes 51 is movable in the third direction to communicate with the first cleaning liquid reservoir 8, thereby respectively inputting the cleaning liquid into the plurality of mixing channels 101 through the first inlets 1011. The second cleaning liquid reservoir 9 is arranged at a side of the second preparation solution reservoir in the fourth direction, so that the plurality of second liquid suction tubes 61 is movable in the fourth direction to communicate with the second cleaning liquid reservoir 9, so as to respectively input cleaning liquid into the plurality of mixing channels 101 through the second inlets 1012. The waste liquid reservoir 10 can be arranged at a side of the product reservoir in the sixth direction, so that the plurality of liquid outlet tubes 71 can move in the sixth direction to communicate with the waste liquid reservoir 10, so that the cleaning liquid flowing out from the outlet 1013 of the mixing channels 101 is transferred to the waste liquid reservoir 10.

In other embodiments, two first cleaning liquid reservoirs 8 may be arranged at two sides of the first preparation solution reservoir in the third direction, or two second cleaning liquid reservoirs 9 may be arranged at two sides of the second preparation solution reservoir in the fourth direction, or two waste liquid reservoirs 10 may be arranged at two sides of the product reservoir in the sixth direction, but the present disclosure is not limited thereto.

For example, in a default initial position, the plurality of first liquid suction tubes 51 may be positioned directly above the first cleaning liquid reservoir 8, the plurality of second liquid suction tubes 61 may be positioned directly above the second cleaning liquid reservoir 9, and the plurality of liquid outlet tubes 71 may be positioned directly above the waste liquid reservoir 10.

The nanoparticle preparation system may further include a first temperature control device (not shown) disposed at the first input well plate 2 and configured to change a temperature of the first preparation solution to a first preparation temperature. The second preparation solution reservoir may further include a second temperature control device (not shown) disposed at the second input well plate 3 and configured to change a temperature of the second preparation solution to a second preparation temperature. For example, the first temperature control device may be arranged at a bottom of the first input well plate 2, and the second temperature control device may be arranged at a bottom of the second input well plate 3. In addition, temperature control devices (not shown) may also be provided at the first cleaning liquid reservoir 8, the second cleaning liquid reservoir 9, the output well plate 4 and the waste liquid reservoir 10, and the present disclosure is not limited thereto. A reaction temperature of the mixing channel 101 can be adjusted by the first temperature control device and the second temperature control device. For example, the first preparation temperature and the second preparation temperature may be the same. The temperature control device can include a heating element, a refrigeration element and a temperature control circuit. The heating element may include a resistor. The heating element and the refrigeration element can be a same semiconductor heating and refrigeration element. The temperature control circuit can include comparators, amplifiers, temperature sensors, etc. to realize closed-loop control of temperature.

The nanoparticle preparation system may further include a controller which is communicatively connected to the first liquid suction device 5, the second liquid suction device 6, the liquid outlet device 7, the temperature control device, etc. to control the operation of the nanoparticle preparation system.

Fig. 8 is a perspective view of the platform 14 of the nanoparticle preparation system according to an embodiment of the present disclosure. The first liquid suction device 5, the second liquid suction device 6, the liquid outlet device 7, the first preparation solution reservoir, the second preparation solution reservoir, the product reservoir, the first cleaning liquid reservoir 8, the second cleaning liquid reservoir 9 and the waste liquid reservoir 10 can all be arranged in the platform 14 to form an integrated nanoparticle preparation system. The platform 14 includes a cabin door and other components.

According to embodiments of the present disclosure, the high-flux microfluidic mixing of preparation solution in multiple rows of solution accommodating wells in the first preparation solution reservoir and the second preparation solution reservoir is realized by the front-back and up-down movement of the whole row of suction tubes and the control of the pump. The preparation and production of high-flux nanoparticles are realized, and the quality of the prepared nanoparticles is stable among batches. Automatic cleaning is realized by arranging the cleaning liquid reservoir 8, the cleaning liquid reservoir 9 and the waste liquid reservoir 10, so that different nanoparticles can be continuously produced, and the production efficiency and flexibility are improved.

### Control method of nanoparticle preparation system

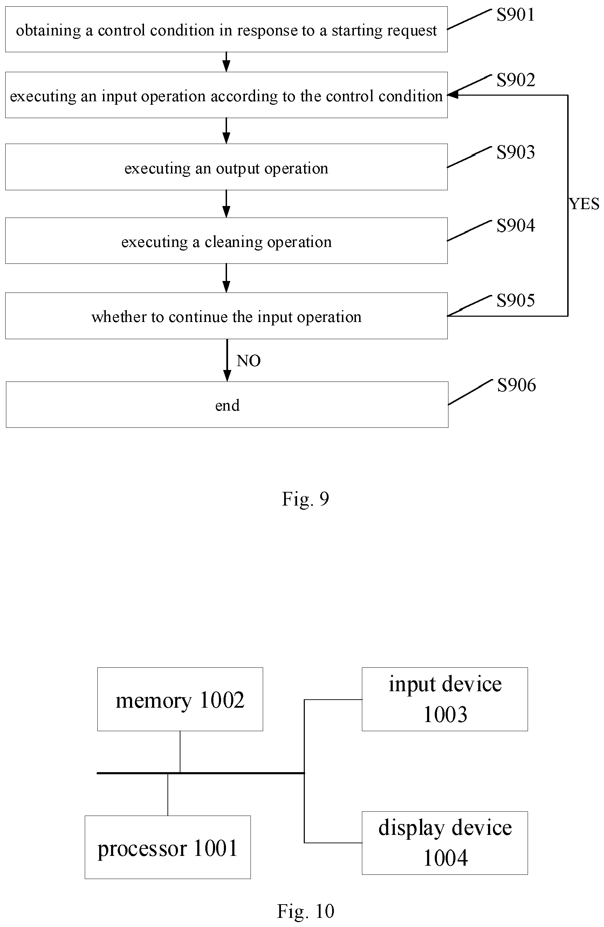

Fig. 9 is a flowchart of a control method of a nanoparticle preparation system according to an embodiment of the present disclosure. As illustrated by Fig. 9, the control method of the nanoparticle preparation system can include:
Step S901, obtaining a control condition in response to a starting request;
Step S902, executing an input operation according to the control condition;
Step S903, executing an output operation;
Step S904, executing a cleaning operation;
Step S905, judging whether to continue the input operation.

If a determination result in step S905 is NO, the process proceeds to step S906, ending the control. If the determination result in step S905 is YES, the process returns to step S902.

For example, the control method can be executed on the nanoparticle preparation system as described above.

In step S901, the control condition may include a mixing speed, a mixing ratio and a preparation temperature. For example, the control method may include determining a first flow rate of the first preparation solution and a second flow rate of the second preparation solution according to the mixing speed and mixing ratio. For example, the first flow rate is the mixing speed multiplied by the mixing ratio of the first preparation solution, and the second flow rate is the mixing speed multiplied by the mixing ratio of the second preparation solution. For example, before executing the input operation, the control method may include controlling the first temperature control device of the first preparation solution reservoir to change the temperature of the first preparation solution to the first preparation temperature according to the preparation temperature; and/or controlling the second temperature control device of the second preparation solution reservoir to change the temperature of the second preparation solution to the second preparation temperature according to the preparation temperature.

In step 902, the input operation may include controlling the plurality of first liquid suction tubes 51 of the first liquid suction device 5 to move above a specific row of the first solution accommodating wells 21 in the third direction relative to the first preparation solution reservoir, and then moving downward so that the plurality of first liquid suction tubes 51 are inserted into the row of the first solution accommodating wells 21 to input the first preparation solution in the row of the first solution accommodating wells 21 into the plurality of mixing channels 101; controlling the plurality of second liquid suction tubes 61 of the second liquid suction device 6 to move above a specific row of the second solution accommodating wells 31 in the fourth direction relative to the second preparation solution reservoir, and then moving downward so that the plurality of second liquid suction tubes 61 are inserted into the row of the second solution accommodating wells 31 to input the second preparation solution in the row of the second solution accommodating wells 31 into the plurality of mixing channels 101. For example, the first preparation solution may be input into the plurality of mixing channels 101 at determined first flow rates, and the second preparation solution may be input into the plurality of mixing channels 101 at determined second flow rates. Herein, the plurality of first flow rates may be different and independently controlled by a plurality of first liquid suction pumps 53. Similarly, the plurality of second flow rates may be different and independently controlled by a plurality of second liquid suction pumps 63.

In step S903, the output operation may include controlling the plurality of liquid outlet tubes 71 of the liquid outlet device 7 to move above a specific row of the product accommodating wells 41 in the sixth direction relative to the product reservoir, and then moving downward so that the plurality of liquid outlet tubes 71 are inserted into the row of the product accommodating wells 41 to guide the products at the outlets 1013 of the plurality of mixing channels 101 into the row of the product accommodating wells 41.

In step S904, the cleaning operation may include controlling a plurality of first liquid suction tubes 51 of the first liquid suction device 5 to move to the first cleaning liquid reservoir 8 in the third direction, so as to input the cleaning liquid in the first cleaning liquid reservoir 8 into the plurality of mixing channels 101 through the first inlets 1011, and controlling the plurality of second liquid suction tubes 61 of the second liquid suction device 6 to move to the second cleaning liquid reservoir 9 in the fourth direction, so as to input the cleaning liquid in the second cleaning liquid reservoir 9 into the plurality of mixing channels 101 through the second inlets 1012. In addition, the cleaning operation also includes controlling the plurality of liquid outlet tubes 71 of the liquid outlet device 7 to transfer the cleaning liquid flowing out of the mixing channels 101 to the waste liquid reservoir 10.

In step S905, judging whether to continue the input operation. For example, if the input operation is executed for a predetermined number of times, it can be judged that the input operation is not executed any more. Alternately, whether to execute the input operation can be judged according to other control condition.

If it is judged that the input operation needs to be continued, returning to step S902, the input operation may include controlling the plurality of first liquid suction tubes 51 of the first liquid suction device 5 to move above a next specific row of the first solution accommodating wells 21 in the third direction relative to the first preparation solution reservoir to input the first preparation solution in the row of the first solution accommodating wells 21 into the plurality of mixing channels 101; and controlling the plurality of second liquid suction tubes 61 of the second liquid suction device 6 to move above a next specific row of the second solution accommodating wells 31 in the fourth direction relative to the second preparation solution reservoir to input the second preparation solution in the row of the second solution accommodating wells 31 into the plurality of mixing channels 101.

Next, in step S903, the output operation may include controlling the plurality of liquid outlet tubes 71 of the liquid outlet device 7 to move above a next specific row of the product accommodating wells 41 in the sixth direction relative to the product reservoir and guiding the products at the outlets 1013 of the plurality of mixing channels 101 into the row of the product accommodating wells 41.

Because the cleaning operation is executed after each input operation and output operation, multiple preparation cycles can be continuously carried out without mutual influence among the multiple preparation cycles.

If it is judged that the input operation is no longer executed, ending the control. For example, the first liquid suction device 5, the second liquid suction device 6 and the liquid outlet device 7 can be returned to the default initial positions.

The above control method can be executed by the controller. In addition, the nanoparticle preparation system may further include a display device and an input device to interact with a user. The display device may include a display. The input device may be a button, a keyboard, or a touch panel (which may be provided on the display device). The user can input the control condition through an input device. The controller can obtain the control condition from the input device.

### Electronic device

Fig. 10 is a schematic diagram of a hardware structure of an electronic device according to an embodiment of the present disclosure. As illustrated by Fig. 10, the electronic device includes at least one processor 1001 and a memory 1002 communicatively connected with the at least one processor 1001. The memory 1002 stores instructions executable by the at least one processor 1001 to enable the at least one processor 1001, which are executed by the at least one processor 1001 to enable the at least one processor 1001 to execute the control method of the nanoparticle preparation system as described above.

The electronic device can be the controller of the nanoparticle preparation system as described above. The electronic device may further include an input device 1003 and a display device 1004.

The processor 1001, the memory 1002, the input device 1003 and the display device 1004 can be connected by a bus or other means. In Fig. 10, bus connection is taken as an example.

The memory 1002, as a non-volatile computer readable storage medium, can be used to store non-volatile software programs, non-volatile computer executable programs and modules, such as program instructions/modules corresponding to the control method of the nanoparticle preparation system in the embodiment of the present disclosure, for example, the method flow illustrated in Fig. 9. The processor 1001 executes various functional applications and data processing by running non-volatile software programs, instructions and modules stored in the memory 1002, that is to say, realizes the control method of the nanoparticle preparation system according to the embodiment of the present disclosure.

The memory 1002 can include a storage program partition and a storage data partition, the storage program partition can store an operating system and application programs required by at least one function; the storage data partition can store data and the like created according to the use of the control method of the nanoparticle preparation system. In addition, the memory 1002 may include a high-speed random access memory and a non-volatile memory, such as at least one disk memory device, a flash memory device, or other non-volatile solid-state memory devices. In some embodiments, the memory 1002 may optionally include memories remotely located with respect to the processor 1001, and these remote memories may be connected to devices that execute the control method of the nanoparticle preparation system through a network. Examples of the above networks include, but are not limited to, the Internet, intranet, local region network, mobile communication network and combinations thereof.

The input device 1003 can receive an input user click, and generate a signal input related to the user setting and function control of the control method of the nanoparticle preparation system. The display device 1004 may include a display or the like.

One or more modules are stored in the memory 1002, and if executed by one or more processors 1001, the control method of the nanoparticle preparation system in any of the above method embodiments is executed.

### Storage medium

At least one embodiment of the present disclosure provides a non-transitory computer readable medium that stores instructions executed by a computing device, and the computing device executes the instructions to cause the computing device to execute the control method of the nanoparticle preparation system as described above.

### Method for preparing nanoparticles

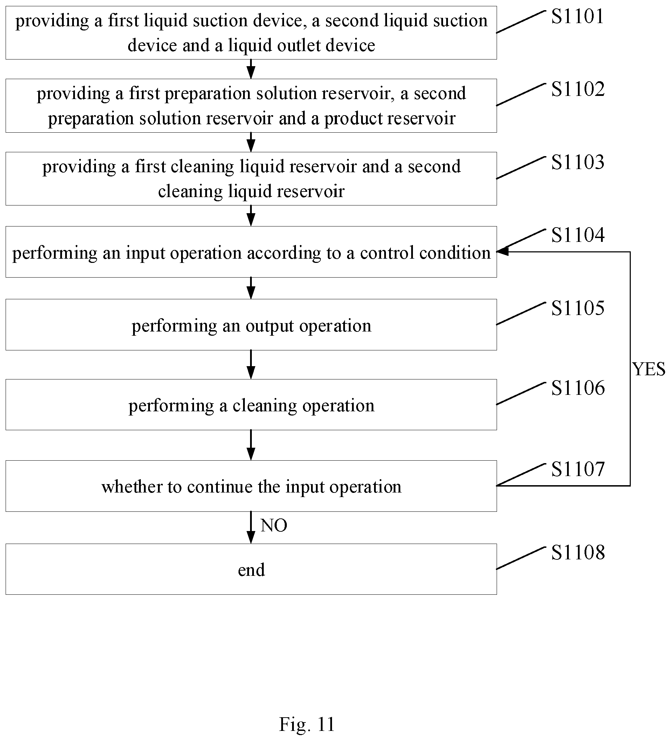

Fig. 11 is a flowchart of a method for preparing nanoparticles according to an embodiment of the present disclosure, which can be carried out, for example, by using the nanoparticle preparation system as described above. As illustrated by Fig. 11, the method includes:
Step S 1101, providing a first liquid suction device 5, a second liquid suction device 6 and a liquid outlet device 7, the first liquid suction device 5 includes a plurality of first liquid suction tubes 51 arranged in rows along a first direction, the second liquid suction device 6 includes a plurality of second liquid suction tubes 61 arranged in rows along a second direction, and the liquid outlet device 7 includes a plurality of liquid outlet tubes 71 arranged in rows along the fifth direction;
Step S1 102, providing a first preparation solution reservoir, a second preparation solution reservoir and a product reservoir, the first preparation solution reservoir includes first solution accommodating wells 21 arranged in an array in the first direction and a third direction perpendicular to the first direction, the second preparation solution reservoir includes second solution accommodating wells 31 arranged in an array in the second direction and a fourth direction perpendicular to the second direction, and the product reservoir includes products accommodating wells 71 arranged in an array in the fifth direction and a sixth direction perpendicular to the fifth direction;
Step S1103, providing a first cleaning liquid reservoir 8 for storing cleaning liquid and a second cleaning liquid reservoir 9 for storing cleaning liquid, the first cleaning liquid reservoir 8 is located at a side of the first preparation solution reservoir in the first direction, and the second cleaning liquid reservoir 9 is located at a side of the second preparation solution reservoir in the first direction;
Step S1104, performing an input operation according to a control condition;
Step S1105, performing an output operation;
Step S1106, performing a cleaning operation;
Step S1107, judging whether to continue the input operation.

If a determination result in step S1107 is NO, the process proceeds to step S1108, ending the control. If the determination result in step S 1107 is YES, the process returns to step S902.

For example, in step S1102, a carrier solution with a specific composition containing a chemical material may be added to the first solution accommodating well 21 of the first preparation solution reservoir. Furthermore, a loaded drug solution containing a target drug may be added to the second solution accommodating well 31 of the second preparation solution reservoir.

For example, different carrier solutions are added in different first solution accommodating wells 21 of the first input well plate 2 as the first preparation solution reservoir, and/or different loaded solution are added in different second solution accommodating wells 31 of the second input well plate 3 as the second preparation solution reservoir. For example, the chemical materials added in the carrier solution are different. Alternately, the components and/or contents of the chemical materials added in the carrier solution are different. For example, the target drugs added in the loaded solution are different. Alternately, the components and/or contents of the target drugs added in the loaded solution are different.

For example, the chemical material is a nanometer material. For example, the chemical material is an organic nanometer material and/or an inorganic nanometer material. The chemical material is not particularly limited by the present disclosure, as long as it is suitable for encapsulating drugs (e.g., nucleic acid drugs, chemical drugs, protein drugs, etc.). In some embodiments, the chemical material is selected from the group consisting of organic nanoparticles (such as lipid nanoparticles, liposomes, polymer nanoparticles, emulsifiers, etc.), inorganic nanoparticles, etc., or their composites. In some embodiments, the chemical material may be a composite of DLin-MC3-DMA/cholesterol/DSPC/DMG-PEG2000, and the ratio of the three can be adjusted according to actual requirements.

For example, the carrier solution contains an organic solvent. That is to say, the organic solvent is used to disperse the chemical material. For example, the organic solvent includes but is not limited to ethanol, ethanol, isopropanol, methanol, acetone, dimethylamide, DMSO, dimethylpolysiloxane and the like.

For example, the loaded solution further contains buffer solution. By adding the buffer solution, it is beneficial to ensure that the target drug is in a stable pH condition, so that it can be better mixed with chemical materials. For example, the buffer solution is aqueous buffer solution, such as sodium citrate solution, sodium acetate solution, phosphate buffer solution, trimethylolaminomethane buffer solution, etc. Further, the aqueous buffer solution may contain hydrochloric acid, acetic acid, citric acid, alkali, glycerin, urea, Tween 20, Tween 80, polyethylene glycol, etc.

For example, the target drugs include nucleic acid drugs, chemical drugs and/or protein drugs. For example, nucleic acid drugs include DNA drugs and/or RNA drugs. DNA drugs include but are not limited to: circular double-stranded DNA plasmid, straight-stranded single-stranded or double-stranded DNA, short single-stranded antisense DNA(ASO), etc. RNA drugs include but are not limited to long single-stranded mRNA, double-stranded mRNA, self-amplifying RNA, circular mRNA (circle RNA), siRNA, microRNA, long non-coding RNA (lncRNA), etc. The chemical drugs can be any small molecule chemical drug in the field, including but not limited to paclitaxel, amphotericin, cytarabine, daunomycin, etc. The protein drugs include but are not limited to insulin, vascular endothelial growth factor, etc. The size and source of the target drug are not particularly limited in the present disclosure, as long as nanoparticles can be prepared by the nanoparticle preparation system of the present disclosure.

For example, the loaded solution contains water and/or organic solvent. That is to say, water and/or organic solvent are used to disperse the target drug. For example, the solvent in the loaded solution is water.

In step S 1104, for example, the control condition includes a mixing speed and a mixing ratio of the first preparation solution and the second preparation solution. The method further includes determining a first flow rate of the first preparation solution and a second flow rate of the second preparation solution according to the mixing speed and the mixing ratio. For example, the control condition includes preparation temperatures. For example, the method further includes changing a temperature of the first preparation solution to a first preparation temperature by using a first temperature control device of the first preparation solution reservoir; and changing a temperature of the second preparation solution to a second preparation temperature by using a second temperature control device of the second preparation solution reservoir. Alternately, the method further includes changing a temperature of the product solution to a product storage temperature by using a third temperature control device of the product reservoir

The input operation includes inputting the first preparation solution into the mixing channels 101 through first inlets 1011 of the mixing channels 101 respectively by using a plurality of first liquid suction tubes 51 of the first liquid suction device 5; and inputting the second preparation solution into the mixing channels 101 through second inlets 1012 of the mixing channels 101 respectively by using a plurality of second liquid suction tubes 61 of the second liquid suction device 6. For example, the input operation includes moving the plurality of first liquid suction tubes 51 of the first liquid suction device 5 in a third direction relative to the first preparation solution reservoir to sequentially input the first preparation solution in the first solution accommodating wells 21 arranged in rows into the plurality of mixing channels 101, respectively; and moving the plurality of second liquid suction tubes 61 of the second liquid suction device 6 in a fourth direction relative to the second preparation solution reservoir to sequentially input the second preparation solution in the second solution accommodating wells 31 arranged in rows into the plurality of mixing channels 101, respectively. For example, the first preparation solution is input to the mixing channel 101 at a first flow rate, and the second preparation solution is input to the mixing channel 101 at a second flow rate.

In step S1105, the output operation includes moving the plurality of liquid outlet tubes 71 of the liquid outlet device 7 in a sixth direction relative to the product reservoir to sequentially guide the products at the outlets 1013 of the plurality of mixing channels 101 to the plurality of product accommodating wells 41 arranged in rows.

In step S1106, the cleaning operation includes moving the plurality of first liquid suction tubes 51 of the first liquid suction device 5 to the first cleaning liquid reservoir 8 in the third direction relative to the first preparation solution reservoir and the first cleaning liquid reservoir 8, and respectively inputting the cleaning liquid in the first cleaning liquid reservoir 8 into the plurality of mixing channels 101; and moving the plurality of second liquid suction tubes 61 of the second liquid suction device 6 to the second cleaning liquid reservoir 9 in the fourth direction relative to the second preparation solution reservoir and the second cleaning liquid reservoir 9, and respectively inputting the cleaning liquid in the second cleaning liquid reservoir 9 into the plurality of mixing channels 101.

In step S1107, judging whether to continue the input operation. For example, if the input operation is performed for a predetermined number of times, it can be judged that the input operation is not performed any more.

In step S1108, the method ends. According to actual requirements, the product solution containing nanoparticles can be treated by means known to those skilled in the art, including but not limited to washing, drying and other steps to obtain dried nanoparticles.

### Examples

As an example, the operation method of the nanoparticle preparation system includes:
Step (1): Starting a machine, opening a cabin door, installing a microfluidic chip 1 on a chip placement position of a platform 14, and upon the operation is completed, closing the cabin door;
Step (2): Preparing liquid A and liquid B in two separate 96-well plates, liquid A is nanoparticles dissolved in alcohol phase;
Step (3): opening the cabin door, respectively putting the 96-well plates filled with liquid A and B at a first input well plate placing position and a second input well plate placing position which are temperature controllable, and putting a groove A and a groove B at a first cleaning liquid reservoir placing position and a second cleaning liquid reservoir placing position, respectively; putting a third 96-well plate at an output well plate placing position to collect the obtained product; putting a groove C at a waste liquid reservoir placing position;
Step (4): Setting the temperatures of the first input well plate placing position and the second input well plate placing position on an operation interface, and setting mixing speed of liquid A and liquid B on the operation interface;
Step (5). Closing the cabin door, pressing the "Start" button, and the machine starts to mix liquid A and liquid B; after all the samples are collected, opening the cabin door, and taking out the 96-well plate at the output well plate placing position, so as to obtain the prepared samples;
Step (6). Pouring out the waste liquid in groove C, and adding a proper volume of cleaning liquid in groove A and groove B;
Step (7). Preparing the sample of the next 96-well plate for collection: preparing liquid A and liquid B in two new separate 96-well plates, the liquid A is nanoparticles dissolved in alcohol phase;
Step (8). Repeating steps (4)-(7) until all samples are collected;
Step (9). After adding a proper volume of cleaning liquid into groove A and groove B, closing the cabin door, selecting "Manual Cleaning" on the operation interface, completing the machine cleaning, and turning off the power supply.

For example, in step (3), the cleaning liquid in groove A is sterile and enzyme-free ultrapure water; the cleaning liquid in groove B is sigma 200-proof pure ethanol.

For example, in step (4), the temperatures of the first input well plate placing position and the second input well plate placing position are set to 25 °C, and the temperature of the output well plate placing position is set to 4 °C.

For example, in step (4), the flow rate of liquid A can be set at 0-120 µL/s, and the flow rate of liquid B can be set at 0-60 µL/s. For nanoparticles, for example, the flow rate of liquid A is 10-20 µL/s, and the flow rate of liquid B is 30-60 µL/s.

The nanoparticle preparation system according to the embodiment of the present disclosure can prepare nanoparticles with a high-flux, and 96 different samples can be prepared in one operation, and the time for preparing the same sample is much shorter than that of a single-flux device, thus greatly improving the efficiency of nanoparticle synthesis. Because there are many factors that need to be regulated in a process of optimizing nanoparticles, the multi-flux system can efficiently regulate many factors. In addition, the nanoparticle preparation system according to the embodiment of the present disclosure breaks through the limitation of single-flux equipment on the preparation amount, which can reduce the preparation amount and avoid the waste of preparation materials.

The nanoparticle preparation system according to the embodiment of the present disclosure adopts a microfluidic system, so that parameters such as volume, rate, proportion and the like in the mixing process can be accurately controlled. Therefore, the size, uniformity and gene encapsulation effect of nanoparticles can be effectively controlled, and the subsequent delivery effect in vitro cells and animals can be improved. Furthermore, the preparation volume of the nanoparticle preparation system according to the embodiment of the present disclosure can be accurately controlled to any volume within 1 mL, and it can simultaneously meet the requirements of in vitro cell experiments and enough animal in vivo experiments, so as to obtain accurate and stable test evaluation results. Moreover, the nanoparticle preparation system according to the embodiment of the present disclosure includes a wide range of application scenarios, such as organic nanoparticles (including but not limited to lipid nanoparticles, liposomes, polymer nanoparticles, emulsifiers, etc.), inorganic nanoparticles, etc., which can all be prepared by the system.

As an example, the nanoparticle preparation system according to an embodiment of the present disclosure is compared with a single-flux microfluidic hybrid system.

### 1. Fast and efficient

### Preparation materials:

Prepare liposome materials (DLin-MC3-DMA/ cholesterol/DSPC/DMG-PEG2000 = 58.5/38.5/10/1.5 mol/mol) and ethanol as carrier solution, prepare nonsense siRNA fragments and 25mM NaOAC buffer solution (pH=4) as loaded solution, and in addition prepare a 96-well plate and several EP tubes. DLin-MC3-DMA is an ionizable cationic liposome; DSPC is 1,2-distearoyl-sn-glycerol -3-choline phosphate (Distearoylphosphatidylcholine); DMG-PEG2000 is dimmyristoyl glycerol-polyethylene glycol 2000 (1,2-dimyristoyl-RAC-glycocero-3-methoxypolyethylene glycol-2000). The sequence of the nonsense siRNA fragment is (5' to 3'):
Sense strand: UUCUCCGAACGUGUCACGUTT
Antisense strand: ACGUGACACGUUCGGAGAATT

### Scheme and method:

1) At the same time and under the same condition, prepare the liposome ethanol solution (carrier solution) and the acidic aqueous solution (loaded solution) of siRNA according to the volume ratio of 3: 1, so that the final total volume of each sample is 200 µL. The total amount of materials prepared are 192 parts;
2) Divide the total amount of the loaded solution and the carrier solution into 192 parts at random, respectively, and use them for nanoparticle preparation in the single-flux system and the nanoparticle preparation system according to the embodiment of the present disclosure, respectively;
3) While the two systems are ready for the operation, start timing respectively. Until the final 96 samples removed the organic solvent are obtained, the timing is completed. Compare the time spent in preparing the same volume (600 µL) and number (96) of samples by the single-flux system and the nanoparticle preparation system according to the embodiment of the present disclosure.

The nanoparticle preparation system according to the embodiment of the present disclosure takes much less time to prepare samples with the same volume and number than that of the single-flux microfluidic hybrid system.

### 2. Sample quality and accuracy

1) Select randomly 20 samples from the samples prepared by the two systems, respectively. Accurately measure the volume of the obtained liquid with a pipette, and calculate the acquisition rate of the final volume, and make a horizontal comparison between samples prepared by the same instrument and a vertical comparison between samples prepared by different instruments;
2) Select randomly 20 samples from the samples prepared by the two systems, respectively. And make a horizontal comparison between samples prepared by the same instrument and a vertical comparison between samples prepared by different instruments in size, dispersion coefficient, electrical property, encapsulation rate and phenotype of nanoparticles in prepared samples.

The nanoparticle preparation system according to the embodiment of the present disclosure is superior to the single- flux microfluidic hybrid system in the acquisition rate of the final volume, the prepared nanoparticle size, dispersion coefficient, electrical property, encapsulation rate and phenotype, and the consistency of each parameter is high.

### 3. Stability between batches

1) Select randomly 20 samples from the samples prepared by the two systems respectively, accurately measure the volume of the obtained liquid with a pipette, calculate the acquisition rate of the final volume and compare among different batches;
2) Select randomly 20 samples from the samples prepared by the two systems respectively, and compare among different batches in size, dispersion coefficient, electrical property, encapsulation rate and phenotype of nanoparticles in the prepared samples.

The nanoparticle preparation system according to the embodiment of the present disclosure is superior to the single-flux microfluidic hybrid system in stability between batches.

### 4. Operation stability of different operators

1) Different operators operate and repeat the above schemes. Randomly selects 20 samples from the samples prepared by the two systems respectively, accurately measure the volume of the obtained liquid with a pipette, calculate the acquisition rate of the final volume, and compare the samples obtained by different operators;
2) Different operators operate and repeat the above schemes. Randomly select 20 samples from the samples prepared by the two systems respectively, and compare the size, dispersion coefficient, electrical property, encapsulation rate and phenotype of nanoparticles in the prepared samples among the samples obtained by different operators.

The nanoparticle preparation system according to the embodiment of the present disclosure is superior to the single-flux microfluidic hybrid system in terms of operation stability of different operators.

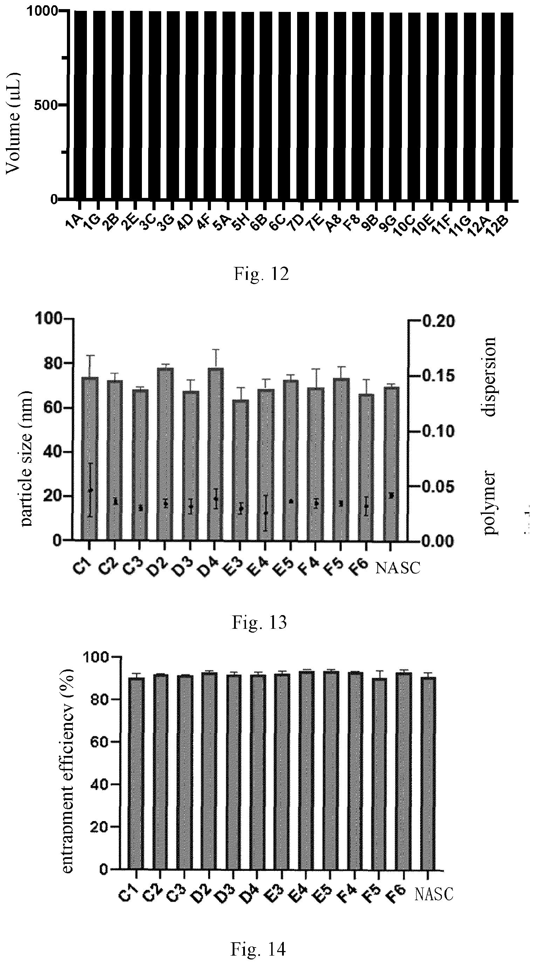

Fig. 12 is a schematic diagram of volume accuracy of Daedalus of a sample prepared by the nanoparticle preparation system according to an embodiment of the present disclosure. In Fig. 12, the vertical axis (Y-axis) is the volume in µL, the horizontal axis represents the number of 24 wells in the 96-well plate as the output well plate, letters represent the serial number in the vertical column, and numbers represent the serial number in the horizontal row. In the nanoparticle preparation system, ethanol and water are used as component A and component B respectively to mix and collect, and the total volume of the target collection is 1 mL. After collection, 24 wells are randomly selected from the 96-well plate as an output well plate for volume measurement. As illustrated by Fig. 12, there is almost no significant difference in sample collection volume among the wells.

Fig. 13 is a schematic diagram of particle size and polymer dispersion index of nanoparticles for encapsulating siRNA (small interfering RNA) prepared by a non-automatic single-flux microfluidic hybrid system and a nanoparticle preparation system according to an embodiment of the present disclosure. In two systems, the same lipid materials and small interfering RNA are both used as component A and component B respectively to be mixed and collected. After the collection is completed, 12 wells are randomly selected from the 96-well plate as the output well plate in the nanoparticle preparation system of the present disclosure to detect the particle size and polymer dispersion index. At the same time, the particle size and polymer dispersion index of the product obtained by the non-automatic single-flux microfluidic hybrid system are detected.

As illustrated by Fig. 13, in the nanoparticle preparation system of the present disclosure, the nanoparticle products obtained from each well include no significant difference in particle size and polymer dispersion index and the uniformity is good. Compared with the nanoparticles obtained by the non-automatic single-flux microfluidic hybrid system, the nanoparticles prepared by the nanoparticle preparation system of the present disclosure include smaller particle size and the same degree of polymer dispersion index.

In Fig. 13, the horizontal axis (X axis) represents the sample numbers in different wells, C1 to F6 represent nanoparticles prepared by the nanoparticle preparation system of the present disclosure, and NASC represents nanoparticles prepared by a non-automatic single-flux microfluidic hybrid system. The left vertical axis (Y axis) is the particle size, and the right vertical axis (Y axis) is the polymer dispersion index; the column indicates the particle size value, and the dot indicates the polymer dispersion index value.

Fig. 14 is a schematic diagram of the encapsulation rate of nanoparticles for encapsulating siRNA (small interfering RNA) prepared by a non-automatic single-flux microfluidic hybrid system and a nanoparticle preparation system according to an embodiment of the present disclosure.

In two systems, the same lipid materials and small interfering RNA are both used as component A and component B respectively to be mixed and collected. After the collection is completed, 12 wells are randomly selected from the 96-well plate as the output well plate in the nanoparticle preparation system of the present disclosure to detect the encapsulation efficiency. At the same time, the encapsulation efficiency of the product obtained by the non-automatic single-flux microfluidic hybrid system is detected.

As illustrated by Fig. 14, in the nanoparticle preparation system of the present disclosure, there is no significant difference in drug encapsulation efficiency of nanoparticle products obtained from each well, and the encapsulation uniformity is good. Compared with nanoparticles obtained by a non-automatic single-flux microfluidic hybrid system, nanoparticles prepared by the nanoparticle preparation system of the present disclosure include high drug encapsulation efficiency.

In Fig. 14, the horizontal axis (X axis) represents the sample numbers in different wells, C1 to F6 represent nanoparticles prepared by the nanoparticle preparation system of the present disclosure, and NASC represents nanoparticles prepared by a non-automatic single-flux microfluidic hybrid system. The vertical axis (y-axis) represents the percentage of package efficiency.

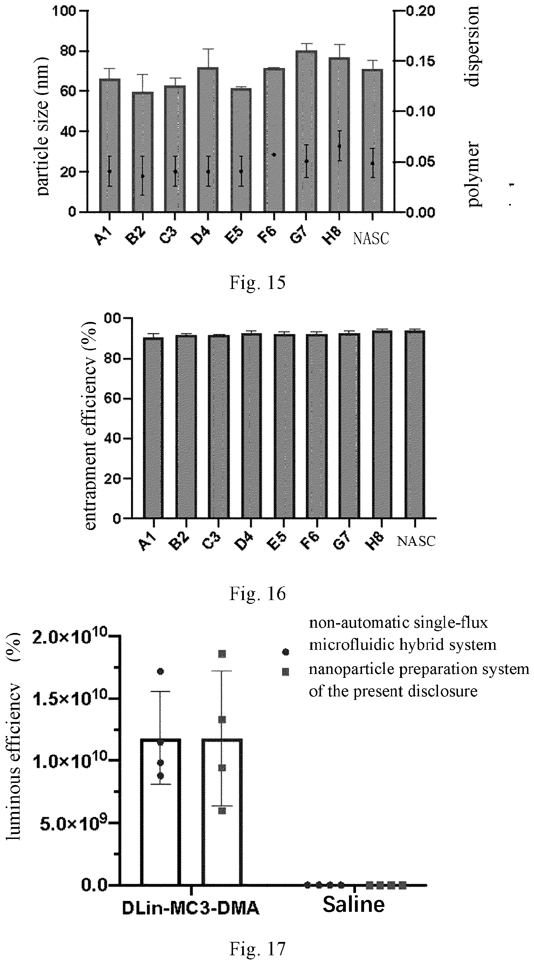

Fig. 15 is a schematic diagram of particle size and polymer dispersion index of nanoparticles for encapsulating mRNA (messenger RNA) prepared by a non-automatic single-flux microfluidic hybrid system and a nanoparticle preparation system according to an embodiment of the present disclosure.

In two systems, the same lipid materials and messenger RNA are both used as component A and component B respectively to be mixed and collected. After the collection is completed, 8 wells are randomly selected from the 96-well plate as the output well plate in the nanoparticle preparation system of the present disclosure to detect the particle size and polymer dispersion index. At the same time, the particle size and polymer dispersion index of the product obtained by the non-automatic single-flux microfluidic hybrid system are detected.

As illustrated by Fig. 15, in the nanoparticle preparation system of the present disclosure, the nanoparticle products obtained from each well include no significant difference in particle size and polymer dispersion index and the uniformity is good. Compared with the nanoparticles obtained by the non-automatic single-flux microfluidic hybrid system, the nanoparticles prepared by the nanoparticle preparation system of the present disclosure include smaller particle size and smaller (i.e. more uniform) polymer dispersion index.

In Fig. 15, the horizontal axis (X axis) represents the sample numbers in different wells, A1 to H8 represent nanoparticles prepared by the nanoparticle preparation system of the present disclosure, and NASC represents nanoparticles prepared by a non-automatic single-flux microfluidic hybrid system. The left vertical axis (Y axis) is the particle size, and the right vertical axis (Y axis) is the polymer dispersion index; the column indicates the particle size value, and the dot indicates the polymer dispersion index value

Fig. 16 is a schematic diagram of the encapsulation rate of nanoparticles for encapsulating mRNA (messenger RNA) prepared by a non-automatic single-flux microfluidic hybrid system and a nanoparticle preparation system according to an embodiment of the present disclosure.

In two systems, the same lipid materials and messenger RNA are both used as component A and component B respectively to be mixed and collected. After the collection is completed, 8 wells are randomly selected from the 96-well plate as the output well plate in the nanoparticle preparation system of the present disclosure to detect the encapsulation efficiency. At the same time, the encapsulation efficiency of the product obtained by the non-automatic single-flux microfluidic hybrid system is detected

As illustrated by Fig. 16, in the nanoparticle preparation system of the present disclosure, there is no significant difference in drug encapsulation efficiency of nanoparticle products obtained from each well, and the encapsulation uniformity is good. Compared with nanoparticles obtained by a non-automatic single-flux microfluidic hybrid system, nanoparticles prepared by the nanoparticle preparation system of the present disclosure include high drug encapsulation efficiency

In Fig. 16, the horizontal axis (X axis) represents the sample numbers in different wells, A1 to H8 represent nanoparticles prepared by the nanoparticle preparation system of the present disclosure, and NASC represents nanoparticles prepared by a non-automatic single-flux microfluidic hybrid system. The vertical axis (y-axis) represents the percentage of package efficiency

Fig. 17 is a schematic diagram of in vivo delivery efficiency of nanoparticles for encapsulating mRNA (messenger RNA) prepared by a non-automatic single-flux microfluidic hybrid system and a nanoparticle preparation system according to an embodiment of the present disclosure.

In two systems, the same DLin-MC3-DMA (lipid material) and firefly luciferase mRNA (messenger RNA) are both used as component A and component B respectively to be mixed and collected. The collected nanoparticles are injected into mice by intravenous injection at the dosage of 0.5 mg/kg, and quantitative luminescence detection is carried out at the same time after 6 hours.

As illustrated by Fig. 17, there is no significant difference in vivo delivery efficiency of nanoparticles obtained by two different systems. Two methods showed extremely high in vivo delivery efficiency.

In Fig. 17, each dot represents the fluorescence value in a mouse, and the histogram is the average value corresponding to each group of dots. DLin-MC3-DMA on the horizontal axis (X axis) represents that each mouse is injected with the same dose and volume of mRNA encapsulation lipid nanoparticles; saline is the negative control, and each mouse is only injected with the same volume of saline. The vertical axis (Y axis) represents the luminous efficiency value, in p/s (photon number per second, proton/s).

As an example, the nanoparticle preparation system according to the embodiment of the present disclosure is compared with the high-flux ethanol injection hybrid system. In the high-flux ethanol injection hybrid system, a robotic arm is used to inject high-flux ethanol to prepare nanoparticles.

### 1. Sample uniformity

### Preparation materials:

Prepare liposome materials (DLin-MC3-DMA/ cholesterol/DSPC/DMG-PEG2000 = 58.5/38.5/10/1.5 mol/mol) and ethanol as carrier solution, prepare firefly luciferase mRNA fragment and 25mM NaOAC buffer solution (pH=4) as the loaded solution, and in addition prepare a 96-well plate and several EP tubes. The nucleotide number of mRNA fragment is 1929.

### Scheme and method:

1) At the same time and under the same condition, prepare liposome ethanol solution (carrier solution) and mRNA acid aqueous solution (loaded solution) according to the volume ratio of 3: 1, and the total amount of prepared materials are 192 parts. The final collection volume of each sample of the two systems is set to its maximum collection volume: 200 µL for the ethanol injection hybrid system and 1 mL for the nanoparticle preparation system according to the embodiment of the present disclosure.
2) Divide the total amount of the carrier solution and the loaded solution into 192 parts at random, respectively, and use them for nanoparticle preparation in the ethanol injection hybrid system and the nanoparticle preparation system according to the embodiment of the present disclosure, respectively;
3) Select randomly 20 samples from the samples prepared by the two systems, respectively. And make a vertical comparison between samples prepared by different instruments in size, dispersion coefficient, electrical property, encapsulation rate and phenotype of nanoparticles in prepared samples.

The nanoparticle preparation system according to the embodiment of the present disclosure is basically the same as the high-flux ethanol injection hybrid system in the acquisition rate of the final volume, the prepared nanoparticle size, dispersion coefficient, electrical property, encapsulation rate and phenotype, and the consistency of each parameter is high.

### 2. Delivery effect

1) Select randomly 20 samples from the samples prepared by the two systems respectively, dilute 10 times with PBS and then ultrafilter to remove the organic solvent;
2) In the above ultrafiltration-completed samples, take 20 µL nanoparticle solution from each sample and carry out cell delivery test in vitro under the same condition. Twenty-four hours after delivery, measure the fluorescence expression intensity of cells by luciferase assay kit, so as to compare the delivery effect of the samples prepared by two systems;
3) Select randomly five samples from the samples prepared by the two systems. Intravenously inject of 50 µL in each mouse and record how many mice can be injected into each prepared sample. Six hours after injection, mice are injected with fluorescein, and placed in a living fluorescence imaging system for imaging. Count the fluorescence value of mouse liver, and analyze the fluorescence intensity, so as to compare the delivery effect of samples prepared by two systems.

The nanoparticle preparation system according to the embodiment of the present disclosure is superior to the high-flux ethanol injection hybrid system in delivery effect.

### 3. Extensive application

Prepare different kinds of chemical nanomaterials: liposome molecule DOPE, polymer PEI, emulsifier Tween 80, and they are prepared into nanoparticles with the same setting parameters by using the ethanol injection hybrid system and the nanoparticle preparation system according to the embodiment of the present disclosure. The size, dispersion coefficient, encapsulation rate and phenotype of nanoparticles in the prepared samples are compared vertically between samples prepared by different instruments.

The nanoparticle preparation system according to the embodiment of the present disclosure includes higher consistency for different chemical nanomaterials than the high-flux ethanol injection hybrid system.

The above-mentioned examples only express several embodiments of the present disclosure, and their descriptions are more specific and detailed, but they should not be construed as limiting the patent scope of the present disclosure. It should be pointed out that for those of ordinary skill in the art, without departing from the concept of the present disclosure, several modifications and improvements can be made, all of which are within the scope of protection of the present disclosure. Therefore, the scope of protection of the patent of the present disclosure shall be subject to the appended claims.

## Claims

1. A nanoparticle preparation system, comprising:
a mixing element, comprising a plurality of mixing channels, wherein the plurality of mixing channels are independent from each other and each of the plurality of mixing channels comprises a first inlet, a second inlet and an outlet;
a first liquid suction device, comprising a plurality of first liquid suction tubes arranged in rows along a first direction and configured to input a first preparation solution into the plurality of mixing channels through first inlets of the plurality of mixing channels, respectively; and
a second liquid suction device, comprising a plurality of second liquid suction tubes arranged in rows along a second direction and configured to input a second preparation solution into the plurality of mixing channels through second inlets of the plurality of mixing channels, respectively.

2. The nanoparticle preparation system according to claim 1, wherein the first liquid suction device further comprises a plurality of first liquid suction pumps respectively connected to the plurality of first liquid suction tubes and configured to input the first preparation solution to corresponding mixing channels through corresponding first liquid suction tubes;
the second liquid suction device further comprises a plurality of second liquid suction pumps respectively connected to the plurality of second liquid suction tubes and configured to input the second preparation solution into corresponding mixing channels through corresponding second liquid suction tubes.

3. The nanoparticle preparation system according to claim 1 or 2, further comprising:
a first preparation solution reservoir, comprising first solution accommodating wells arranged in an array in the first direction and a third direction perpendicular to the first direction for storing the first preparation solution;
a second preparation solution reservoir, comprising second solution accommodating wells arranged in an array in the second direction and a fourth direction perpendicular to the second direction for storing the second preparation solution;
the plurality of first liquid suction tubes are movable in the third direction relative to the first preparation solution reservoir to sequentially input the first preparation solution in a plurality of rows of first solution accommodating wells into the plurality of mixing channels, respectively,
the plurality of second liquid suction tubes are movable in the fourth direction relative to the second preparation solution reservoir to sequentially input the second preparation solution in a plurality of rows of second solution accommodating wells into the plurality of mixing channels, respectively.

4. The nanoparticle preparation system according to claim 3, wherein the first direction is the same as the second direction, the third direction is the same as the fourth direction,
the plurality of first liquid suction tubes and the plurality of second liquid suction tubes are configured to move synchronously in the third direction and the fourth direction.

5. The nanoparticle preparation system according to claim 3 or 4, wherein a number of the plurality of first liquid suction tubes and a number of the first solution accommodating wells in the first direction are the same as a number of the plurality of mixing channels, and
a number of the second liquid suction tubes and a number of the second solution accommodating wells in the second direction are the same as the number of the mixing channels.

6. The nanoparticle preparation system according to any of claims 1-5, further comprising:
a liquid outlet device, comprising a plurality of liquid outlet tubes arranged in rows along a fifth direction and configured to respectively guide nanoparticle products at outlets of the plurality of mixing channels out of the plurality of mixing channels.

7. The nanoparticle preparation system according to claim 6, further comprising:
a product reservoir, comprising product accommodating wells arranged in an array in the fifth direction and a sixth direction perpendicular to the fifth direction;
wherein the liquid outlet device is movable in the sixth direction relative to the product reservoir to sequentially guide the nanoparticle products at the outlets of the plurality of mixing channels into a plurality of rows of product accommodating wells.

8. The nanoparticle preparation system according to any of claims 3-7, further comprising:
a first cleaning liquid reservoir for storing cleaning liquid; and
a second cleaning liquid reservoir for storing cleaning liquid,
wherein the first cleaning liquid reservoir is arranged at a side of the first preparation solution reservoir in the third direction, so that the plurality of first liquid suction tubes can move in the third direction to be communicated with the first cleaning liquid reservoir, thereby respectively inputting the cleaning liquid into the plurality of mixing channels through the first inlets;
the second cleaning liquid reservoir is arranged at a side of the second preparation solution reservoir in the fourth direction, so that the plurality of second liquid suction tubes can move in the fourth direction to be communicated with the second cleaning liquid reservoir, thereby respectively inputting the cleaning liquid into the plurality of mixing channels through the second inlets.

9. The nanoparticle preparation system according to any of claims 3-8, wherein the plurality of first liquid suction tubes can move in a vertical direction relative to the first preparation solution reservoir,
the plurality of second liquid suction tubes can move in the vertical direction relative to the second preparation solution reservoir,
the vertical direction is perpendicular to the first direction, the second direction, the third direction and the fourth direction.

10. The nanoparticle preparation system according to any of claims 1-9, wherein the mixing element comprises at least one microfluidic chip, and the plurality of mixing channels are arranged in the at least one microfluidic chip.

11. The nanoparticle preparation system according to any of claims 3-10, wherein the first preparation solution reservoir further comprises a first temperature control device configured to change a temperature of the first preparation solution to a first preparation temperature, and
the second preparation solution reservoir further comprises a second temperature control device configured to change a temperature of the second preparation solution to a second preparation temperature.

12. A method for preparing nanoparticles by using a nanoparticle preparation system, comprising the following steps:
providing a mixing element, comprising a plurality of mixing channels, wherein the plurality of mixing channels are independent from each other and each of the plurality of mixing channels comprises a first inlet, a second inlet and an outlet;
providing a first liquid suction device, comprising a plurality of first liquid suction tubes arranged in rows along a first direction;
providing a second liquid suction device comprising a plurality of second liquid suction tubes arranged in rows along a second direction; and
performing an input operation according to a control condition, wherein the input operation comprises:
inputting a first preparation solution into the plurality of mixing channels through first inlets of the plurality of mixing channels by using the plurality of first liquid suction tubes of the first liquid suction device; and
inputting second preparation solution into the plurality of mixing channels through second inlets of the plurality of mixing channels by using the plurality of second liquid suction tubes of the second liquid suction device.

13. The method according to claim 12, further comprising:
providing a first preparation solution reservoir comprising first solution accommodating wells arranged in an array in the first direction and a third direction perpendicular to the first direction;
providing a second preparation solution reservoir comprising second solution accommodating wells arranged in an array in the second direction and a fourth direction perpendicular to the second direction;
providing a first cleaning liquid reservoir at a side of the first preparation solution reservoir in a first direction, wherein the first cleaning liquid reservoir stores cleaning liquid;
providing a second cleaning liquid reservoir at a side of the second preparation solution reservoir in a first direction, wherein the second cleaning liquid reservoir stores cleaning liquid; and
performing a cleaning operation,
wherein the input operation comprises:
using the plurality of first liquid suction tubes of the first liquid suction device to move in a third direction relative to the first preparation solution reservoir, and sequentially inputting the first preparation solution in a plurality of rows of first solution accommodating wells into the plurality of mixing channels; and
using the plurality of second liquid suction tubes of the second liquid suction device to move in a fourth direction relative to the second preparation solution reservoir, and sequentially inputting the second preparation solution in a plurality of rows of second solution accommodating wells into the plurality of mixing channels.
wherein the cleaning operation comprises:
moving the plurality of first liquid suction tubes of the first liquid suction device to the first cleaning liquid reservoir in a third direction relative to the first preparation solution reservoir and the first cleaning liquid reservoir, and inputting the cleaning liquid in the first cleaning liquid reservoir into the plurality of mixing channels, respectively; and
moving the plurality of second liquid suction tubes of the second liquid suction device to the second cleaning liquid reservoir in a fourth direction relative to the second preparation solution reservoir and the second cleaning liquid reservoir, and inputting the cleaning liquid in the second cleaning liquid reservoir into the plurality of mixing channels, respectively,
wherein the cleaning operation is performed after each input operation

14. The method according to claim 12 or 13, wherein the control condition comprises a mixing speed and a mixing ratio of the first preparation solution and the second preparation solution;
the method comprises:
determining a first flow rate of the first preparation solution and a second flow rate of the second preparation solution according to the mixing speed and the mixing ratio;
wherein in the input operation:
using the plurality of first liquid suction tubes of the first liquid suction device to input the first preparation solution into the mixing channels at the first flow rate through the first inlets of the plurality of mixing channels, respectively; and
using the plurality of second liquid suction tubes of the second liquid suction device to input the second preparation solution into the mixing channels at the second flow rate through the second inlets of the plurality of mixing channels, respectively.

15. The method according to any of claims 12-14, further comprising:
providing a liquid outlet device comprising a plurality of liquid outlet tubes arranged in rows along a fifth direction;
providing a product reservoir comprising a plurality of product accommodating wells arranged in an array in the fifth direction and a sixth direction perpendicular to the fifth direction; and
performing an output operation, wherein the output operation comprises:
using the plurality of liquid outlet tubes of the liquid outlet device to move in the sixth direction relative to the product reservoir, and sequentially guiding nanoparticle products at the outlets of the plurality of mixing channels to a plurality of rows of product accommodating wells.
